# EUROPEAN PATENT SPECIFICATION

(11) **EP 2 852 314 B1**
(45) Date of publication and mention of the grant of the patent: **24.04.2019**
(21) Application number: 13725192.2
(22) Date of filing: 21.05.2013
(51) Int. Cl.: A61L 9/015, A61L 9/20, A61L 9/22, A47K 10/48

(54) **COMBINED HAND DRYER AND AIR STERILISER APPARATUS**
KOMBINIERTER HANDTROCKNER UND LUFTSTERILISATOR
SÈCHES-MAINS ET STÉRILISATEUR D'AIR COMBINÉ

(30) Priority: 21.05.2012 GB 201208891
(43) Date of publication of application: 01.04.2015
(73) Proprietor: Heat Outdoors Limited, Bishops Stortford, Hertfordshire CM17 0DR (GB)
(72) Inventor: LEVY, Steve, Bishops Stortford Hertfordshire CM17 0DR (GB)
(74) Representative: Sewell, Adrian David
(86) International application number: PCT/GB2013/051331
(87) International publication number: WO 2013/175202

(56) References cited:
- GB-A- 2 399 010
- GB-A- 2 407 038

## Description

### Field of the Invention

The present invention relates to air sterilising apparatus and to hand dryers. It is particularly applicable to a combination room and air sterilising/deodorising unit and a hand dryer unit combined inside one wall mounted electrical appliance, and the synergy of such a combination.

### Background to the invention

In modern washrooms or public conveniences electric hand dryers are usually provided. These offer a number of advantages over roller towels or paper towels for drying hands after washing. They are particularly hygienic and can cut costs by as much as 90% compared to using paper towels. They require little maintenance compared to paper towels, which must be replenished regularly and the paper waste removed on a regular basis.

There are a number of different types of electric hand dryer on the market currently, but these all have some common features to their operation. Ambient air is drawn into the hand drier, usually passed over a heating element, and dispensed at high velocity from the dryer at the user's hands. It follows that these hand dryers require an electrical supply. GB2407038A discloses a combination hand dryer/air steriliser apparatus having the features of the preamble of claim 1.

One persistent problem found in washrooms and public conveniences is that of unpleasant odours. In washrooms and toilets smells and unpleasant odours can be created by some or all of the following: bacteria, viruses, mould, faecal matter and volatile organic compounds (VOCs) carried in the air and present on surfaces. In addition, bacteria can multiply very quickly and are continually being brought into a washroom by new users. These odours can be exacerbated by and distributed by the large volumes of air circulated by electric hand dryers.

Various known cleaning methods can be used to reduce these odours. Without wishing to be constrained by any particular theory or theories, these include:
(a) **Ozone,** this obliterates bacteria, viruses and fungi, and removes odour from the air.
(b) **Photoplasma,** a type of energised oxygen, eliminates the reproductive capability of micro-organisms and reduces the number of micro-organisms, which disinfects the air.
(c) **Negative ions,** decrease the total concentration of harmful chemicals in the air.
(d) **Deep-UV-light,** sterilises micro-organisms that pass through the unit.
(e) **Photo Catalytic Oxidation** process destroys volatile organic compounds, such as methane, on the surface material.

These processes are well known to those skilled in the art of air sterilisers and air purification systems. It will be understood that one or more of these various methods and processes can be used in any combination as required. It is also possible, and in fact it is desirable, that all these processes are used together.

Existing air purification units are generally positioned in washrooms at high level, out of reach of the general public. They usually rely on a small fan, as in the case of units described in US2011/0033346 (Bohlen and Bozoo). These small fans require frequent cleaning to remove debris so these units require regular maintenance.

In all cases, these air purification units require an electrical supply, which may be difficult or expensive to install, or both. In this context the terms 'air purification unit' and 'air sterilisation unit' are used interchangeably.

It is an object of the present invention to overcome or at least mitigate some or all of the problems outlined above. The invention provides a combined hand dryer and air purification unit in a single housing, utilising a single electrical supply. The invention also uses a convection air stream to distribute air flow and thus does away with the need for a dedicated fan.

### Summary of the Invention

According to the present invention there is provided a combination hand dryer/air steriliser apparatus comprising:
a housing unit housing a hand dryer unit and an air sterilisation unit, the housing unit having an air intake opening, a first air outlet opening, and a second air outlet opening for a first airflow to pass from said air intake opening to said first air outlet opening and through said hand dryer unit, and for a second airflow to pass from said air intake opening to said second air outlet opening and through said air sterilisation unit;
said hand dryer unit comprising:
   (a) a duct having a duct input opening and a duct output opening;
   (b) a fan unit disposed in fluid communication with said duct for generating said first airflow from said air intake opening to said first air outlet opening via said duct input opening and said duct output opening;
   (c) a heater disposed in fluid communication with said duct for heating said first airflow;
   (d) a control circuit for energising said fan unit and said heater in response to an actuation signal and being configured to energise said air sterilisation unit on a substantially permanent basis, even when the fan unit and heater unit are not energised;
   characterised in that:
   - the air sterilisation unit is located outside of said first airflow generated by the fan unit,
   - the air intake opening is disposed in a lower portion of the housing unit and the second air outlet opening is disposed in an upper portion of the housing unit such that, when the hand dryer unit is inoperative, said second airflow operating the sterilisation unit is generated by convection currents only, and
   - the apparatus further comprises an air deflector adapted to deflect a proportion of air, prior to the air entering the input opening of the hand dryer unit, towards the air sterilization unit and away from the hand dryer unit.

Preferably the air sterilisation unit operates using photocatalytic oxidation and one or more of the following processes:
ozone generation;
photoplasma generation;
negative ion generation; and
deep-UV-light.

Preferably the air sterilisation unit comprises a catalyst and a plurality of UV lamps. Preferably the catalyst comprises titanium dioxide. Preferably the catalyst comprises a plurality of titanium dioxide plates.

The air deflector, which causes a portion of air otherwise intended for the hand dryer to be directed into the sterilisation unit, further enhances the performance of the air sterilisation unit.

Preferably the air deflected by the air deflector is deflected over UV lamps in the air sterilisation unit.

Preferably the air deflector comprises a vane projecting into the airflow, prior to the air entering the duct input opening of the hand dryer unit.

Preferably the vane incorporates a plurality of apertures such that only a proportion of the airflow striking the vane is directed towards the air sterilisation unit.

Preferably the air sterilisation unit produces one or more of the sterilising agents selected from the group comprising: Ozone, Photoplasma, Negative Ions, UV Light and PCO (Photocatalytic Oxidation) products. The sterilisation unit thus operates using known technologies. It will therefore be appreciated that any suitable air sterilisation technology, known or yet to be discovered, can be utilised in the present invention.

### Brief Description of the Drawings

Preferred embodiments of the present invention will now be described, by way of example only, with reference to the following drawing in which:
Figure 1 illustrates a schematic diagram of the contents of a first embodiment of the dryer/steriliser;
Figures 2 and 3 illustrate rear and front elevational views of the sterilizer unit; and
Figure 4 illustrates a schematic diagram of the contents of a first embodiment of the dryer/steriliser.

### Description of the Preferred Embodiments

The invention will now be described by way of example only. These are currently the best ways known to the applicant of putting the invention into practice, but they are not the only way that this can be done.

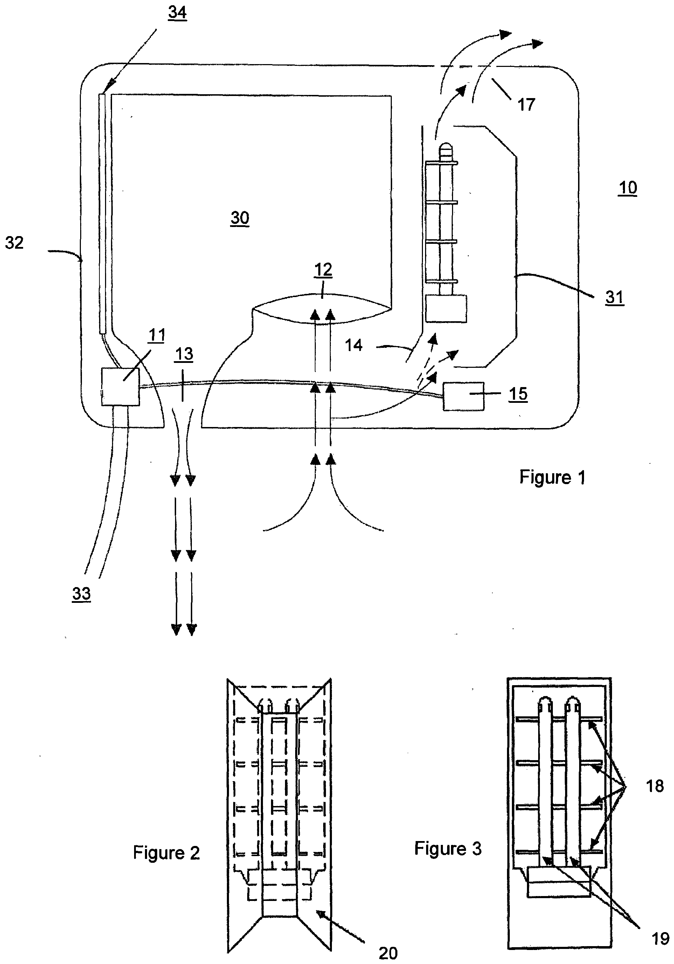

Figure 1 shows in diagrammatic and schematic form the internal components of a combination hand dryer/air sterilizer apparatus 10. It should be noted that the drawings, and the various components within those drawings, are not necessarily to scale. As shown in Figure 1 the apparatus includes a hand dryer unit 30 and a photocatalytic oxidation (PCO) unit or air sterilizer unit 31. These are mounted on a common base 32 and housed within one housing (not shown).

The hand dryer in this example is of a common format in which air is drawn in through an air intake, or air input opening, by a fan unit and the air flow generated is ducted by a duct over a heater unit (not shown), heated, and the resultant warm airflow ejected at speed from an air outlet, or air output opening 13. Both the air input opening and the air output opening are on the underside of the housing when the base plate and housing are mounted on a wall in a conventional manner.

It will, however, be appreciated that different designs of hand dryer, such as those where the hands are inserted into a drying chamber and withdrawn slowly, could equally well be used in this invention. Such a hand dryer is described in US2007/0079524 (Sato et al).

Returning to Figure 1, an electrical power source supplies power to a power stabilizer 11, which in turn feeds a hand dryer PCB 34. A control circuit controls the power supply to a fan and to a heater, which are only activated when the motion or presence of hands are detected under the air output opening 13.

Thus far a conventional hand dryer unit has been described. In addition to the hand dryer unit, housed within the same housing is a PCO/air sterilisation unit 31. This comprises a convection chamber 20 which houses a plurality of titanium dioxide (TiO₂) coated plates 18. In the context of this description the term "plurality" has the meaning one or more. Passing through these plates is a dual wavelength UV lamp in an 'H' configuration 19. The light or electromagnetic radiation from these lamps shining on the TiO₂ coated plates causes photocatalytic oxidation, as well as generating ozone. The technology associated with photocatalytic oxidation is well known and any suitable oxidation apparatus can be used to generate negative ion oxygen species and/or ozone.

It will be understood that other types and arrangements of sterilising unit or units could be used. Different configurations of lamps or just a single lamp could be used, as could a different arrangement of plates. Although titanium dioxide is a particularly useful catalyst to use in this application, other catalysts could be used in addition or instead.

The PCO unit obtains its electrical supply from the same source 33 as the hand dryer by way of a lamp choke 15. This electrical supply operates independently of the hand dryer function, such that the sterilising unit operates at all times that there is an electrical supply to the unit. Typically these units are left on 24 hours per day to maintain a clean, odourless environment in the washroom or wherever the unit is located.

During a first mode of action, when no one is drying their hands and the hand dryer is inoperative, the PCO unit operates by convection currents only, without any fan assistance. Ions pass out of the housing through exit 17 in convected air. However, when the hand dryer is in operation the PCO unit receives a boost. A deflector flap or vane 14 extends into the airflow stream near the hand dryer air intake 12 and this directs a portion of the intake air stream through the air sterilisation unit 31. This diverted airflow is directed generally over the UV lamp(s), and increases both the amount of ions released by the apparatus and the extent to which those ions are distributed around the space around the apparatus. This creates the boost effect.

The diverter flap can take a number of different forms and may include a plurality of apertures in the flap such that only a proportion of the air striking the flap is diverted from its normal path into the hand dryer unit.

From the foregoing description it will be appreciated that there are a number of unexpected synergies as a result of combining the hand dryer unit with an air sterilisation unit. Firstly the air movements set up by using a hand dryer, particularly a high velocity hand dryer, increase the flow of convected air through the air sterilisation unit for a considerable time, even after the hand dryer has stopped operating.

Secondly, a proportion of the input air to the hand dryer unit is diverted through the air sterilisation unit. This further increases its effectiveness, and at just the time when this increased effectiveness is needed. That is, when someone has used the washroom or public convenience services.

Thirdly, no separate electrical power supply is required for the air sterilisation unit, as would be the case if the sterilisation unit was a stand alone item.
In summary, the invention provides a dual purpose product combining a room and air sterilising/deodorising apparatus and a hand dryer inside one wall mounted electrical appliance. The sterilising/deodorising apparatus has no moving parts and creates airflow by a combination of compression of the airspace and thermal convection, together in this embodiment with utilisation of diverted air, diverted from the hand dryer inlet into the air sterilisation unit.

The principle objective is to deodorise the air and sterilize all exposed surfaces in the room.

The hand dryer part is a modern high velocity dryer utilising a powerful motor/fan to drive air via an electric heating element to an outlet nozzle or nozzles. The principle objective is to dry wet hands.

The sterilising/deodorising module within the unit is designed to deodorize and sterilize both air and all exposed surfaces in rooms. The unit has no moving parts and generally plasma, ozone or ion distribution is by thermal convection. The unit uses dual waveband H lamps 185nm/254nm low voltage 9 Watts, the combined waveband breaks down the harmful ozone and a TiO₂ catalyst also breaks down the ozone by a process known as photocatalytic oxidation (PCO). These combined technologies oxidize and degrade organic contaminants (VOC's), bacteria, germs and virus throughout the room.

It will however be appreciated that the invention is not dependant on the use of any one particular air sterilising technology. Any technology or technologies that create the necessary sterilising species and/or ozone can be used in this application and it need not depend on the type of photocatalytic technology described above.

When the hand dryer is operated, as well as the normal air flow needed to dry hands efficiently, a negative air flow zone is generated creating an air vortex driving higher speed air though the sterilisation unit at higher velocity than normal. This helps to break up the normal air flow in the room and distribute the plasma more fully and at greater speed. This effect is not present, and is not possible to achieve, in conventional standalone air sterilisation units.

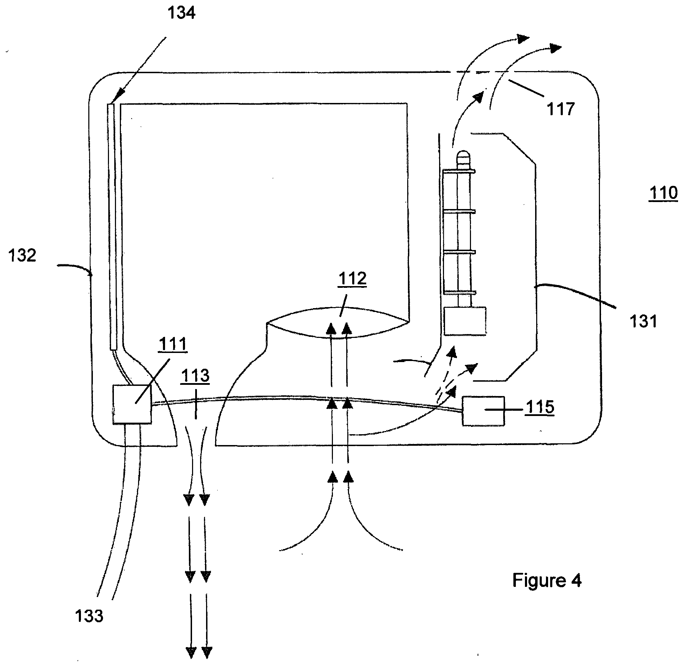

Figure 4 shows in diagrammatic and schematic form the internal components of a second embodiment of the combination hand dryer/air sterilizer apparatus 110. Like reference numerals in this Figure are used to refer to like parts appearing in earlier Figures. It should be noted that the drawings, and the various components within those drawings, are not necessarily to scale. As shown in Figure 4 the apparatus includes a hand dryer unit 130 and a photocatalytic oxidation (PCO) unit or air sterilizer unit 131. These are mounted on a common base 132 and housed within one housing (not shown).

An electrical power source supplies power to a power stabilizer 111 , which in turn feeds a hand dryer PCB 134. A control circuit controls the power supply to a fan and to a heater, which are only activated when the motion or presence of hands are detected under the air output opening 113. The hand dryer includes an air intake 112.

In addition to the hand dryer unit, housed within the same housing is the PCO/air sterilisation unit 131.

As in the previous embodiment, it will be understood that other types and arrangements of sterilising unit or units could be used. Again, different configurations of lamps or just a single lamp could be used, as could a different arrangement of plates.

The PCO unit obtains its electrical supply from the same source 133 as the hand dryer by way of a lamp choke 115. This electrical supply operates independently of the hand dryer function, such that the sterilising unit operates at all times that there is an electrical supply to the unit. Typically these units are left on 24 hours per day to maintain a clean, odourless environment in the washroom or wherever the unit is located.

During a first mode of action, when no one is drying their hands and the hand dryer is inoperative, the PCO unit operates by convection currents only, without any fan assistance. Ions pass out of the housing through an exit 117 in convected air. However, when the hand dryer is in operation the PCO unit still receives a boost. This boost effect arises notwithstanding the lack of a deflector flap or vane extending into the airflow stream near the hand dryer air intake. While the PCO unit is not in the path of the air flow generated by the fan unit (and in particular the warmed airflow ejected at speed from the air outlet), the resultant fluid flow of the air within the housing when the fan unit is in operation, with air drawn in into the fan unit through the air intake 112, is found to boost air in the vicinity of the PCO unit towards and through the exit 117.

Some of the key features are therefore as follows:-
1. The sterilisation unit is mounted vertically inside the hand dryer case as per Figures 1 and 4.
2. When the 185 nm lamp generates ozone 0 2+hv1-0-0 in the restricted volume of the unit chamber it has to go somewhere and expands leaving a vacuum behind. This combined with the heat generated from the lamp triggers the thermal convection to distribute the plasma generated by the combined technology.
3. The air diffusers set at right angles to the base plate have holes at strategic points to allow the air to pass through them, but are designed to slow the air movement, increasing the dwell time and maximizing the treatment of the air in the chamber.
4. The 254 nm waveband treats all the air in the chamber with germicidal irradiation and assists in decomposing the Ozone before exiting the unit according to the following formula 0 3+Hv2-0 2+0
5. The 254 nm waveband will also sterilize any bacteria and viruses passing through the chamber. The (aluminum) Heterogeneous Catalyst also acts as an eye shield and is coated with Ti02 which absorbs light and treats the air sequentially creating hydroxyl radicals (-0H is formed) as the air exits.
6. The air leaving the unit - Plasma Quatro, is a gas energized and contains activated oxygen, ozone and superoxide ions. This combination is much more efficient than either ozone or UV working alone.

## Claims

1. A combination hand dryer/air steriliser apparatus (10,110) comprising:
a housing unit housing a hand dryer unit (30,130) and an air sterilisation unit (31,131), the housing unit having an air intake opening, a first air outlet opening, and a second air outlet opening (17,117) for a first airflow to pass from said air intake opening to said first air outlet opening and through said hand dryer unit (30,130), and for a second airflow to pass from said air intake opening to said second air outlet opening (17,117) and through said air sterilisation unit (31,131);
said hand dryer unit (30) comprising:
(a) a duct having a duct input opening (12,112) and a duct output opening (13,113);
(b) a fan unit disposed in fluid communication with said duct for generating said first airflow from said air intake opening to said first air outlet opening via said duct input opening (12,112) and said duct output opening (13,113);
(c) a heater disposed in fluid communication with said duct for heating said first airflow;
(d) a control circuit for energising said fan unit and said heater in response to an actuation signal and being configured to energise said air sterilisation unit (31,131) on a substantially permanent basis, even when the fan unit and heater unit are not energised;
**characterised in that**:
- the air sterilisation unit (31,131) is located outside of said first airflow generated by the fan unit,
- the air intake opening is disposed in a lower portion of the housing unit and the second air outlet opening (17,117) is disposed in an upper portion of the housing unit such that, when the hand dryer unit (30,130) is inoperative, said second airflow operating the sterilisation unit (31,131) is generated by convection currents only, and
- the apparatus further comprises an air deflector (14) adapted to deflect a proportion of air, prior to the air entering the input opening of the hand dryer unit (30,130), towards the air sterilization unit (31,131) and away from the hand dryer unit (30,130).

2. A combination hand dryer/air steriliser apparatus according to Claim 1, wherein the air sterilization unit (31,131) operates using photocatalytic oxidation and one or more of the following processes:
ozone generation;
photoplasma generation;
negative ion generation; and
deep-UV-light.

3. A combination hand dryer/air steriliser apparatus according to Claim 1 or Claim 2, wherein the air sterilization unit (31,131) comprises a catalyst and plurality of UV lamps (19).

4. A combination hand dryer/air steriliser apparatus according to Claim 3, wherein the catalyst comprises titanium dioxide.

5. A combination hand dryer/air steriliser apparatus according to Claim 3, wherein the air deflected by the air deflector (14) is deflected over the UV lamps in the air sterilization unit (31,131).

6. A combination hand dryer/air steriliser apparatus according to any preceding claim, wherein the air deflector (14) comprises a vane projecting into the airflow, prior to the air entering the duct input opening (12,112) of the hand dryer unit (30,130).

7. A combination hand dryer/air steriliser apparatus according to Claim 6, wherein the vane incorporates a plurality of apertures such that only a proportion of the airflow striking the vane is directed towards the air sterilization unit (31,131).

## Patentansprüche

1. Kombinations-Handtrockner/Luftsterilisatorvorrichtung (10, 110) die Folgendes umfasst:
eine Gehäuseeinheit, die eine Handtrocknereinheit (30, 130) und eine Luftsterilisationseinheit (31, 131) unterbringt, wobei die Gehäuseeinheit eine Luftansaugöffnung, eine erste Luftauslassöffnung und eine zweite Luftauslassöffnung (17, 117) aufweist, damit ein erster Luftstrom von der Luftansaugöffnung zu der ersten Luftauslassöffnung und durch die Handtrocknereinheit (30, 130) gelangt und ein zweiter Luftstrom von der Luftansaugöffnung zu der zweiten Luftauslassöffnung (17, 117) und durch die Luftsterilisationseinheit (31, 131) gelangt;
wobei die Handtrocknereinheit (30) Folgendes umfasst:
(a) einen Kanal mit einer Kanaleingangsöffnung (12, 112) und einer Kanalausgangsöffnung (13, 113);
(b) eine Gebläseeinheit, die in Fluidverbindung mit dem Kanal angeordnet ist, um den ersten Luftstrom von der Luftansaugöffnung zu der ersten Luftauslassöffnung über die Kanaleingangsöffnung (12, 112) und die Kanalausgangsöffnung (13, 113) zu erzeugen;
(c) eine Heizung, die in Fluidverbindung mit dem Kanal angeordnet ist, um den ersten Luftstrom zu erwärmen;
(d) einen Steuerstromkreis zum Versorgen der Gebläseeinheit und der Heizung mit Energie als Reaktion auf ein Betätigungssignal, und die dazu konfiguriert ist, die Luftsterilisationseinheit (31, 131) auf einer im Wesentlichen dauerhaften Basis mit Energie zu versorgen, selbst wenn die Gebläseeinheit und die Heizungseinheit nicht mit Energie versorgt werden;
**dadurch gekennzeichnet, dass**:
- sich die Luftsterilisationseinheit (31, 131) außerhalb des von der Gebläseeinheit erzeugten ersten Luftstroms befindet,
- die Luftansaugöffnung in einem unteren Abschnitt der Gehäuseeinheit angeordnet ist und die zweite Luftauslassöffnung (17, 117) in einem oberen Abschnitt der Gehäuseeinheit angeordnet ist, sodass, wenn die Handtrocknereinheit (30, 130) funktionslos ist, der die Sterilisationseinheit (31, 131) betreibende zweite Luftstrom nur durch Konvektionsströmungen erzeugt wird, und
- die Vorrichtung weiter eine Luftumlenkvorrichtung (14) umfasst, die dazu angepasst ist, bevor die Luft in die Eingangsöffnung der Handtrocknereinheit (30, 130) eintritt, einen Anteil von Luft in Richtung der Luftsterilisationseinheit (31, 131) und von der Handtrocknereinheit (30, 130) weg umzulenken.

2. Kombinations-Handtrockner/Luftsterilisatorvorrichtung nach Anspruch 1, wobei die Luftsterilisationseinheit (31, 131) unter Verwendung von photokatalytischer Oxidation und einem oder mehreren der folgenden Prozesse arbeitet:
Ozonerzeugung;
Photoplasmaerzeugung;
Erzeugung negativer Ionen; und
Tief-UV-Licht.

3. Kombinations-Handtrockner/Luftsterilisationsvorrichtung nach Anspruch 1 oder Anspruch 2, wobei die Luftsterilisationseinheit (31, 131) einen Katalysator und eine Vielzahl von UV-Lampen (19) umfasst.

4. Kombinations-Handtrockner/Luftsterilisatorvorrichtung nach Anspruch 3, wobei der Katalysator Titandioxid umfasst.

5. Kombinations-Handtrockner/Luftsterilisatorvorrichtung nach Anspruch 3, wobei die von der Luftumlenkvorrichtung (14) umgelenkte Luft über die UV-Lampen in der Luftsterilisatoreinheit (31, 131) umgelenkt wird.

6. Kombinations-Handtrockner/Luftsterilisatorvorrichtung nach einem der vorangehenden Ansprüche, wobei die Luftumlenkvorrichtung (14) eine in den Luftstrom ragende Leitplatte, bevor die Luft in die Kanaleingangsöffnung (12, 112) der Handtrocknereinheit (30, 130) eintritt, umfasst.

7. Kombinations-Handtrockner/Luftsterilisatorvorrichtung nach Anspruch 6, wobei die Leitplatte eine Vielzahl von Löchern beinhaltet, sodass nur ein Anteil des auf die Leitplatte auftreffenden Luftstroms in Richtung der Luftsterilisationseinheit (31, 131) gelenkt wird.

## Revendications

1. Un appareil sèche-mains/stérilisateur d'air combiné (10, 110) comprenant :
une unité logement logeant une unité sèche-mains (30, 130) et une unité de stérilisation d'air (31, 131), l'unité logement possédant une ouverture d'admission d'air, une première ouverture de sortie d'air et une deuxième ouverture de sortie d'air (17, 117) destinée au passage d'un premier flux d'air à partir de ladite ouverture d'admission d'air vers ladite première ouverture de sortie d'air et au travers de ladite unité sèche-mains (30, 130), et destinée au passage d'un deuxième flux d'air à partir de ladite ouverture d'admission d'air vers ladite deuxième ouverture de sortie d'air (17, 117) et au travers de ladite unité de stérilisation d'air (31, 131),
ladite unité sèche-mains (30) comprenant :
(a) un conduit possédant une ouverture d'entrée de conduit (12, 112) et une ouverture de sortie de conduit (13, 113),
(b) une unité ventilateur disposée en communication fluidique avec ledit conduit de façon à générer ledit premier flux d'air à partir de ladite ouverture d'admission d'air vers ladite première ouverture de sortie d'air par l'intermédiaire de ladite ouverture d'entrée de conduit (12, 112) et de ladite ouverture de sortie de conduit (13, 113),
(c) un dispositif de chauffage disposé en communication fluidique avec ledit conduit destiné au chauffage dudit premier flux d'air,
(d) un circuit de commande destiné à alimenter en énergie ladite unité ventilateur et ledit dispositif de chauffage en réponse à un signal d'actionnement et étant configuré de façon à alimenter en énergie ladite unité de stérilisation d'air (31, 131) sur une base sensiblement permanente, même lorsque l'unité ventilateur et l'unité dispositif de chauffage ne sont pas alimentés en énergie,
**caractérisé en ce que** :
- l'unité de stérilisation d'air (31, 131) est installée à l'extérieur dudit premier flux d'air généré par l'unité ventilateur,
- l'ouverture d'admission d'air est disposée dans une partie inférieure de l'unité logement et la deuxième ouverture de sortie d'air (17, 117) est disposée dans une partie supérieure de l'unité logement de sorte que, lorsque l'unité sèche-mains (30, 130) est non opérationnelle, ledit deuxième flux d'air actionnant l'unité de stérilisation (31, 131) soit généré par des courants de convection uniquement, et
- l'appareil comprend en outre un déflecteur d'air (14) adapté de façon à dévier une proportion d'air, avant l'entrée de l'air par l'ouverture d'entrée de l'unité sèche-mains (30, 130), vers l'unité de stérilisation d'air (31, 131) et à l'écart de l'unité sèche-mains (30, 130).

2. Un appareil sèche-mains/stérilisateur d'air combiné selon la Revendication 1, où l'unité de stérilisation d'air (31, 131) fonctionne au moyen d'un procédé d'oxydation photocatalytique et d'un ou de plusieurs des procédés suivants :
génération d'ozone,
génération de photoplasma,
génération d'ions négatifs, et
lumière ultraviolette profonde.

3. Un appareil sèche-mains/stérilisateur d'air combiné selon la Revendication 1 ou 2, où l'unité de stérilisation d'air (31, 131) comprend un catalyseur et une pluralité de lampes UV (19).

4. Un appareil sèche-mains/stérilisateur d'air combiné selon la Revendication 3, où le catalyseur comprend du dioxyde de titane.

5. Un appareil sèche-mains/stérilisateur d'air combiné selon la Revendication 3, où l'air dévié par le déflecteur d'air (14) est dévié par dessus les lampes UV dans l'unité de stérilisation d'air (31, 131).

6. Un appareil sèche-mains/stérilisateur d'air combiné selon l'une quelconque des Revendications précédentes, où le déflecteur d'air (14) comprend une pâle faisant saillie vers le flux d'air, avant l'entrée de l'air dans l'ouverture d'entrée de conduit (12, 112) de l'unité sèche-mains (30, 130).

7. Un appareil sèche-mains/stérilisateur d'air combiné selon la Revendication 6, où la pâle comprend une pluralité d'ouvertures de sorte qu'uniquement une proportion du flux d'air frappant la pâle soit dirigée vers l'unité de stérilisation d'air (31, 131).
